Europäisches Patentamt

European Patent Office

Office européen des brevets

(19)

(11) Veröffentlichungsnummer: **0 211 014**
**B1**

(12) **EUROPÄISCHE PATENTSCHRIFT**

(45) Veröffentlichungstag der Patentschrift: **26.09.90**

(21) Anmeldenummer: **86900708.8**

(22) Anmeldetag: **13.01.86**

(86) Internationale Anmeldenummer:
**PCT/AT86/00003**

(87) Internationale Veröffentlichungsnummer:
**WO 86/04086 17.07.86 Gazette 86/17**

(51) Int. Cl.⁵: **C 12 N 1/20** // A61K39/104,
C07K15/00

(54) VERFAHREN ZUR SUBMERSEN ZÜCHTUNG VON PSEUDOMONAS AERUGINOSA-BAKTERIENSTÄMMEN.

(30) Priorität: **14.01.85 AT 73/85**

(43) Veröffentlichungstag der Anmeldung:
**25.02.87 Patentblatt 87/09**

(45) Bekanntmachung des Hinweises auf die
Patenterteilung:
**26.09.90 Patentblatt 90/39**

(84) Benannte Vertragsstaaten:
**AT BE CH DE FR GB IT LI LU NL SE**

(56) Entgegenhaltungen:
GB-A-1 201 638
US-A-3 987 164

Infection and Immunity, Band 35, Nr. 1, Januar
1982; T.C. Montie et al.: "Flagellar Preparations
from Pseudomonas aeruginosa: Isolation and
Characterization", Seiten 281-288

Chemical Abstracts, Band 103, Nr. 25, 23.
Dezember 1985, Columbus, Ohio (US); R.
Craven et al.:"Regulation of Pseudomonas
aeruginosa chemotaxis by the nitrogen source,
Seite 510, Zusammenfassung Nr. 210772z

(73) Patentinhaber: IMMUNO Aktiengesellschaft für
chemisch-medizinische Produkte
Industriestrasse 72
A-1220 Wien (AT)

(72) Erfinder: MONTIE, Thomas, C. University of
Tennessee Dep.of
Microbiology Walters Life Science Building
Knoxville,TN 37996-0854 (US)
Erfinder: DORNER, Friedrich
Peterlinigasse 17
A-1230 Wien (AT)
Erfinder: McDONEL, James, L.
814 Cottage Grove Avenue
South Bend, IN 46616 (US)
Erfinder: MUNDT, Wolfgang
Florianigasse 57
A-1080 Wien (AT)

(74) Vertreter: Wolfram, Gustav, Dipl.-Ing.
Schwindgasse 7 P.O. Box 205
A-1041 Wien (AT)

(56) ·Entgegenhaltungen:
Chemical Abstracts, Band 90, Nr. 15, 9 April
1979, Columbus, Ohio (US) R. Moulton et al.:
"Chemotaxis by Pseudomonas aeruginosa",
siehe Seite 326, Zusammenfassung 117911x

**Beschreibung**

Die Erfindung betrifft ein Verfahren zur submersen Züchtung von Pseudomonas aeruginosa-Bakterienstämmen aus der taxonomischen Familie der Pseudomonadaceae.

Bakterium-Pseudomonas aeruginosa ist ein opportunistischer, pathogener Keim, der häufig bei Krankenhausinfektionen auftritt, hauptsächlich bei Patienten mit geschwächter Immunabwehr, wie bei Verbrennungspatienten, bei Personen, die an cystischer Fibrose leiden oder organische Fehlfunktionen aufweisen, und bei Tumorpatienten. Antibiotika sind gegen Pseudomonasinfektionen infolge des Auftretens von Resistenzen nur beschränkt wirksam, weshalb man bemüht ist, immunologische Methoden zur Bekämpfung von Infektionen durch Pseudomonas aeruginosa anzuwenden.

Infektionen können durch eine Vielzahl von Stämmen ausgelöst werden, die O-Gruppenantigene und H-Antigene produzieren. Gemäß dem H-Antigenschema nach Ansorg (Zbl. Bakt. Hyg. I. Abt. Orig. A 242, 228—238 (1978)) wird unter Verwendung der indirekten Immunfluoreszenztechnik bei Pseudomonas aeruginosa ein komplexes Flagella-Antigen a mit den Partialantigenen $a_0$, $a_1$, $a_2$, $a_3$, $a_4$ und ein uniformes Flagella-Antigen b differenziert. Die Partialfaktoren $a_0$—$a_4$ sind unabhängige Determinanten, so daß ein flagellares Antigenschema mit mehreren H-Typen resultiert. O-Gruppen und H-Typ zeigen freie Kombinationen.

Es ist bereits bekannt, zur Prophylaxe gegen Pseudomonasinfektionen Pseudomonasvakzine herzustellen, wobei als Ausgangsmaterialien entweder Pseudomonas aeruginosa-Bakterienmasse selbst und/oder Kulturfiltrate verwendet werden, die unter Züchtung der Mikroorganismen auf Oberflächenkulturen oder submers in komplexen Nährmedien gewonnen wurden. Bei diesen komplexen Nährmedien wurden neben einer Kohlenstoff- und Energiequelle (meist Kohlenhydrate) und essentiellen Nährsalzen verschiedenste Extrakte und/oder Hydrolysate tierischer, mikrobieller oder pflanzlicher Proteine (sogenannte Peptone) verwendet. Derartige Nährlösungssupplemente sind in ihrer genauen Zusammensetzung nicht definiert und zudem von Charge zu Charge variabel. Sie enthalten neben Aminosäuren auch unvollständig abgebaute Proteinbruckstücke und undefinierte Komplexe derselben und dienen im wesentlichen zur Deckung des Aminosäure- und Wuchsstoffbedarfes. Kulturüberstände sind deshalb immer reich an Substanzen nicht-bakteriellen Ursprunges, was den Nachteil hat, daß für die Bereitung eines Flagella-Antigens von Pseudomonas aeruginosa immer mehrere der Kultivierungsstufe nachzustellende Trennstufen notwendig sind, um ein immunogen wirksames Flagella-Antigen soweit wie möglich von aus dem Nährmedium stammenden Verunreinigungen zu befreien.

Ein weiterer Nachteil der bisherigen chargen- bzw. "batch"-weise durchgeführten Züchtungsverfahren, besteht darin, daß die Bakterien vom Zeitpunkt ihrer Inokulation bis zum Zeitpunkt ihrer Abtrennung aus der Kultur einem Zustand der kontinuierlichen physiologischen Änderung unterworfen sind. Das auslösende Moment für diese physiologische und daher funktionelle Differenzierung der bakteriellen Biomasse ist durch das Zellwachstum selbst und der damit ursächlich bedingten zeitlichen Veränderung der Zusammensetzung des Nährmediums bedingt. Die sichtbare Folge dieser spontanen physiologischen Differenzierung ist allein schon an den verschiedenen Phasen der Wachstumskurve einer "batch"-Kultur zu ermessen. Als Konsequenz der beschriebenen direkten Beziehung zwischen der physiologischen Aktivität der Zellmasse einerseits und ihrem Umgebungsmilieu andererseits ist damit auch eine bevorzugte Anreicherung verschiedener intra- und extrazellulärer Stoffwechselprodukte je nach Wachtsumsphase und Differenzierungsphase verbunden. Kulturüberstände einer "batch"-Kultur enthalten deshalb eine integrierte Mischung aller zeitlich über einzelne Differenzierungsphasen gebildeten Stoffwechselprodukte.

Die Erfindung bezweckt die Vermeidung dieser Schwierigkeiten und Nachteile und stellt sich die Aufgabe, ein submerses Züchtungsverfahren von Pseudomonas aeruginosa-Bakterienstämmen zu schaffen, welches mit wesentlich besserer Ausbeute als bisher eine bakterielle Biomasse zur Gewinnung von Antigenen liefert, deren physiologischer Zustand während der Gesamtdauer der Kultivierung konstant bleibt; bei welchem ein synthetisches Nährmedium verwendet wird, aus dem keine zusätzlichen Verunreinigungen durch Autoklavieren entstehen; und welches eine erleichterte chemische Aufarbeitung der bakteriellen Biomasse zu Flagella-Antigen ermöglicht.

Diese Aufgabe wird erfindungsgemäß dadurch gelöst, daß die folgenden H-Typ-Antigene produzierendem Stämme

| Stamm | H-Typ | |
|---|---|---|
| 170001 (ATCC 33350) | — | b |
| M-2 (ATCC 33349) | — | b |
| 5940*) | — | $a_0, a_2$ |
| 5939*) | — | $a_0, a_3$ |
| 5933*) | — | $a_0, a_1, a_2$ |
| 1210 (ATCC 33354) | — | $a_0, a_1, a_2$ |
| 170018 (ATCC 3356) | — | $a_0, a_3, a_4$ |

*) Die Stämme werden in ZbL. Bakt. Hyg., I. Abt. Orig. A242, 228—238 (1978) beschrieben und können von der Collection de l'Institut Pasteur (Paris) bezogen werden.

in einem wässerigen, antigenfreien und proteinfreien Nährmedium, welches eine Stickstoffquelle und Mineralsalze sowie Succinat oder Harnstoff als Kohlenstoffquelle und als limitierendes Substrat enthält, gezüchtet werden.

Die genannte Kohlenstoffquelle hat sich in besonderem Maß als wachstumsfördernd erwiesen.

Für die Stämme 170001, 5940, 5939, 5933 und 170018 ist die Bezeichnung nach Ansorg verwendet. Die Zuordnung der untypisierten Stämme M-2 und 1210 zum entsprechenden H-Serotyp erfolgte aufgrund von Vergleichsuntersuchungen der Molekulargewichte und serologischen Kreuzreaktionen durch Montie et al.

Vorzugsweise wird die Züchtung der Stämme im kontinuierlicher Weise durchgeführt.

Nach einer weiteren bevorzugten Arbeitsweise kann die Züchtung auf turbidostatischem Weg erfolgen, wobei der Sauerstoffgehalt im Nährmedium 5 bis 20%, vorzugsweise etwa 10%, beträgt, die Zelldichte auf $2-3 \times 10^9$ Zellen/ml gehalten und eine Verdünnungsrate des Substrates durch Zuführung von frischem Nährmedium auf 0,1 My bis 0,3 My eingehalten wird.

Die erfindungsgemäße Züchtung der Pseudomonas aeruginosa-Bakterienstämme erfolgt unter Agitation und Belüftung einer "offenen Kultur" in solcher Weise, daß sie zum maximalen Wachstum und Ausbildung der Flagellen veranlaßt werden. Die Zufuhr des Nährsubstrates wird in Abhängigkeit von der Wachstumsrate der Kultur bzw. vom Gleichgewichtszustand, den die Kultur erreicht hat, vergenommen. Die Regelung bzw. Steuerung der Zufuhr von frischem Nährmedium erfolgt in Abhängigkeit von einem dem Wachstum der Kultur proportionalen Parameter, vorzugsweise der zeitlichen Veränderung der Zelldichte. Es können jedoch auch andere Parameter, die der Entwicklung der Kultur proportional sind, wie Atmungsaktivität, $CO_2$-Produktion, Änderung der Wasserstoffionenkonzentration, Änderung der Kohlenstoffquellenkonzentration, Respirationsquotient, Stickstoffverbrauch, Sauerstoffverbrauch und Wärmetönung, herangezoen werden. Weiters kann die Regelung der Zufuhr von frischem Nährmedium in Abhängigkeit von der Menge des gebildeten Antigens erfolgen.

Bei der erfindungsgemäßen kontinuierlichen Züchtungsmethode wird gleichzeitig mit der Zufuhr von frischem Nährmedium Kulturflüssigkeit aus dem Züchtungsgefäß abgezogen, so daß sich zwischen dem Wachstum und der physiologischen Aktivität, insbesondere der Bildung von Zellen einerseits und dem Umgebungsmilieu der Zellen anderseits, ein genau definierbarer und jederzeit reproduzierbarer Fließgleichgewichtszustand einstellt, welcher auf praktisch zeitlich unbegrenzte Dauer erhalten werden kann.

Als Nährmedium wird ein synthetisches proteinfreies Medium verwendet, welches ausschließlich chemisch sowie physikochemisch qualitativ und quantitativ erfaßbare Bestandteile enthält. Geeignet ist eine anorganische Nährlösung, die alle essentiellen Elemente enthält.

Beim bevorzugten erfindungsgemäßen Verfahren sind für die fermentative Produktion der Keime sowohl das chemostatische Prinzip der kontinuierlichen Kultur, bei welchem die physiologische Aktivität der Kultur durch ein limitierendes Substrat, vorzugsweise der Kohlenstoff- und Energiquelle, reguliert wird, wie auch das turbidostatische Prinzip der kontinuierlichen Kultur, bei welchem die Verdünnungsgeschwindigkeit (My) und damit die physiologische Aktivität der Kultur extern über einen Wachstumsparameter, vorzugsweise über die augenblickliche Zelldichte der Kultur, reguliert wird, anwendbar.

Vorzugsweise wird die turbidostatische kontinuierliche Kultivierung angewendet.

Um ein maximales Bakterienwachstum zu fördern, sind auch die Temperaturen bzw. der pH-Wert von Bedeutung; so soll zweckmäßig während der Züchtung eine Temperatur von 20 bis 35°C, vorzugsweise 30°C, und ein pH-Wert von 6,5 bis 7,5 vorzugsweise 7,0, eingehalten werden.

Im einzelnen wird das erfindungsgemäße Verfahren bei turbidostatischer Arbeitsweise in einem Fermentor durchgeführt, indem zunächst das Nährmedium mit einem Bakterienstamm beimpft und unter Einhaltung der angegebenen Bedingungen gearbeitet wird, bis die angegebene Zelldichte von $2-3\times10^9$ Zellen/ml in der logarithmischen Endphase erreicht wird. Sobald dies der Fall ist, wird aus dem Fermentor Baktiensuspension kontinuierlich abgezogen und durch frische Nährlösung kontinuierlich ersetzt. Die Verdünnungsrate soll zweckmäßig nicht mehr als 0,3 My, vorzugsweise 0,1 bis 0,2 My, betragen.

Unter den angegebenen Bedingungen werden nach Erreichen des Fließgleichgewichtszustandes 1,5 bis 2,0 g/l nasse Zellen erhalten, was einem Trübungswert der Suspension vor der Zentrifugierung bei 590 nm nach der Methode von Tyndall von 0,5 bis 0,7 entspricht.

Das erfindungsgemäße Verfahren wird durch das folgende Beispiel näher erläutert:

Beispiel:

Es wurde ein Nährlösung folgender Zusammensetzung verwendet:

| | |
|---|---|
| Dinatriumsuccinat | 4,05 g/l, |
| Dikaliummonohydrogenphosphat | 7 g/l, |
| Kaliumdihydrogenphosphat | 3 g/l, |
| Ammoniumhydrogenphosphat | 1 g/l, |
| Magnesiumsulfat · 7 $H_2O$ | 0,05 g/l, |
| Eisen(III)chlorid | 0,0025 g/l, |

der pH-Wert auf 7,0 eingestellt und die Temperatur auf 30°C gebracht. Die Nährlösung wurde mit dem Stamm Pseudomonas aeruginosa M-2 beimpft und in einen 15 l Fermentor eingebracht. Während einer Fermentationsdauer von 96 Stunden wurden 140 l des Nährmediums durchgesetzt, wobei der Luftdurchsatz 10% $pO_2$ (Sauerstoffpartialdruck) betrug. Die Verdünngsrate betrug 0,1 My. Die Zelldichte nach Erreichen des Fließgleichgewichtszustandes betrug $2-3\times10^9$ Zellen/ml.

Unter den gleichen Bedingungen wurden die Stämme 170001 und 1210 kultiviert, wobei in der folgenden Tabelle die Kulturbedingungen, Verdünnungsrate, Sauerstoffpartialdruck in % und Zellenausbeute in g/l Naßgewicht nach zweimaliger Zentrifugation bei 15000×g ersichtlich ist. Weiters sind die Trübungswerte der Suspension vor der Zentrifugierung angegeben.

| | Kulturbedingungen | | Zellausbeute | |
|---|---|---|---|---|
| Stamm | Verdünnungs-rate | Sauerstoff-gehalt | Trübungswert bei 590 nm d=1 cm | g/l nasse Zellen |
| 170001 | 0,1 My | 10% $pO_2$ | 0,6—0,67 | 1,7 |
| M-2 | 0,1 My | 10% $pO_2$ | 0,6—0,7 | 1,6—1,7 |
| 1210 | 0,1 My | 10% $pO_2$ | 0,56—0,65 | 1,5—1,6 |
| 1210 | 0,2 My | 10% $pO_2$ | 0,6 | 1,5—1,6 |

**Patentansprüche**

1. Verfahren zur submersen Züchtung von Pseudomonas aeruginosa-Bakterienstämmen aus der taxonomischen Familie der Pseudomonadaceae, dadurch gekennzeichnet, daß die folgenden H-Typ-Antigene produzierenden Stämme

| Stamm | | H-Typ |
|---|---|---|
| 170001 | — | b |
| M-2 | — | b |
| 5940 | — | $a_0, a_2$ |
| 5939 | — | $a_0, a_3$ |
| 5933 | — | $a_0, a_1, a_2$ |
| 1210 | — | $a_0, a_1, a_2$ |
| 170018 | — | $a_0, a_3, a_4$ |

in einem wässerigen, antigenfreien und proteinfreien Nährmedium, welches eine Stickstoffquelle und Mineralsalze sowie Succinat oder Harnstoff als Kohlenstoffquelle und als Limitierendes Substrat enthält, auf kontinuierliches Weise und auf turbidostatischem Weg gezüchtet werden, wobei der Sauerstoffgehalt im Nährmedium 5 bis 20%, vorzugsweise etwa 10%, beträgt, die Zelldichte auf $2-3 \times 10^9$ Zellen/ml gehalten und eine Verdünnungsrate des Substrates durch Zuführung von frischem Nährmedium auf 0,1 My bis 0,2 My eingehalten wird.

2. Verfahren nach Anspruch 1, dadurch gekennzeichnet, daß während der Züchtung eine Temperatur von 20 bis 35°C, vorzugsweise 30°C, und ein pH-Wert von 6,5 bis 7,5 vorzugsweise 7,0, eingehalten werden.

**Revendications**

1. Procédé pour la culture submergée de souches bactériennes de Pseudomonas aeuriginosa de la famille taxonomique des pseudomonadecées, caractérisé en ce que l'on cultive de manière continue et suivant un procédé turbidostatique les souches suivantes productrices d'antigènes de type H

| Souche | | Type H |
|---|---|---|
| 170001 | — | b |
| M-2 | — | b |
| 5940 | — | $a_0, a_2$ |
| 5939 | — | $a_0, a_3$ |
| 5933 | — | $a_0, a_1, a_2$ |
| 1210 | — | $a_0, a_1, a_2$ |
| 170018 | — | $a_0, a_3, a_4$ |

dans un milieu nutritif aqueux exempt d'antigènes et de protéines, qui contient une source d'azote et des sels minéraux ainsi qu'un succinate ou de l'urée comme source de carbone et comme substrat limitatif, la teneur en oxygène dans le milieu nutritif étant de 5 à 20%, de préférence d'environ 10%, la densité de cellules étant maintenue à $2-3 \times 10^9$ cellules/ml et en maintenant une vitesse de dilution du substrat par l'apport de milieu nutritif frais à 0,1—0,3 My.

2. Procédé selon la revendication 1, caractérisé en ce que l'on maintient pendant la culture une température de 20 à 35°C, de préférence 30°C et un pH de 6,5 à 7,5, de préférence 7,0.

# EP 0 211 014 B1

**Claims**

1. Method of submersely growing Pseudomonas aeruginosa bacterial strains of the taxonomic family of Pseudomonadaceae, characterized in that the folowing H-type antigen producing strains

| strain | | H-type |
|--------|---|--------|
| 170001 | — | b |
| M-2 | — | b |
| 5940 | — | $a_0, a_2$ |
| 5939 | — | $a_0, a_3$ |
| 5933 | — | $a_0, a_1, a_2$ |
| 1210 | — | $a_0, a_1, a_2$ |
| 170018 | — | $a_0, a_3, a_4$ |

are grown in an aqueous nutrient medium in continuous manner and turbidostatically, said aqueous nutrient medium being free from antigens and free from proteins and containing a nitrogen source and mineral salts as well as succinate or urea as a carbon source and as limiting substrate, the oxygen content in the nutrient medium amounting to from 5 to 20%, preferably approximately 10%, a cellular density of $2—3 \times 10^9$ cells/ml being maintained and a substrate dilution rate of from 0.1 μ to 0.3 μ being maintained by supplying fresh nutrient medium.

2. Method according to Claim 1, characterized in that during growing a temperature of from 20 to 35°C, preferably 30°C, and a pH of from 6.5 to 7.5, preferably 7.0, are maintained.

6